# EUROPEAN PATENT SPECIFICATION

(11) **EP 4 364 760 B1**
(45) Date of publication and mention of the grant of the patent: **16.09.2026**
(21) Application number: 22832994.2
(22) Date of filing: 23.06.2022
(51) Int. Cl.: A61L 29/08, A61L 29/12, A61L 31/10, A61L 31/12, C08F 8/32, C08F 220/28, C08F 220/56

(54) **ELONGATED MEDICAL DEVICE, AND METHOD FOR PRODUCING ELONGATED MEDICAL DEVICE**
LÄNGLICHE MEDIZINISCHE VORRICHTUNG UND VERFAHREN ZUR HERSTELLUNG EINER LÄNGLICHEN MEDIZINISCHEN VORRICHTUNG
DISPOSITIF MÉDICAL ALLONGÉ ET PROCÉDÉ DE PRODUCTION DE DISPOSITIF MÉDICAL ALLONGÉ

(30) Priority: 30.06.2021 JP 2021108552
(43) Date of publication of application: 08.05.2024
(73) Proprietor: ASAHI INTECC CO., LTD., Seto-shi, Aichi 489-0071 (JP)
(72) Inventor: FUTAMI, Soichi, Seto-shi, Aichi 489-0071 (JP); TAKATA, Yumi, Seto-shi, Aichi 489-0071 (JP); YAMAMOTO, Shinpei, Hakusan-shi, Ishikawa 924-0057 (JP)
(74) Representative: Winter, Brandl - Partnerschaft mbB
(86) International application number: PCT/JP2022/025084
(87) International publication number: WO 2023/276847

(56) References cited:
- JP-A- 2011 505 179
- JP-A- 2011 515 515
- JP-A- 2012 533 660
- JP-A- 2019 058 466

## Description

### TECHNICAL FIELD

The present disclosure relates to a coated elongated medical device.

### BACKGROUND ART

Conventionally, medical devices provided with various hydrophilic coating films are known (see for example JP 2011 - 505 179 A, JP 2012 - 533 660 A, JP 2019 - 058 466 A, JP 2011 - 515 515 A). For example, as an elongated medical device such as a catheter or a guide wire that is used by being inserted into a living body, an elongated medical device is known in which coating is performed using a hydrophilic coating agent to impart the surface of the elongated medical device with lubricity. Specifically, for example, in WO 2015 / 137 259 A1, a configuration is disclosed in which a hydrophilic epoxy resin coating film is formed on a medical device using a hydrophilic polymer having a hydrophilic monomer and a reactive functional group such as an epoxy group. Furthermore, in JP 2011 - 110 392 A, a guide wire is disclosed in which a thin film primer containing a functional group is formed on a base material, and a hydrophilic compound is immobilized on the base material by reacting the functional group of the thin film primer with the hydrophilic compound.

### SUMMARY OF INVENTION

### TECHNICAL PROBLEM

However, for example, when forming a coating film using an epoxy resin on a base material made of metal, and a hydrophilic substance is mixed with the coating film material in the manner described in WO 2015 / 137 259 A1, the inventors of the present application have found a new problem that obtaining sufficient adhesion can be relatively difficult. In such a case, in order to obtain more sufficient adhesion, it is necessary to individually provide a layer containing the hydrophilic substance and a layer for adhering the hydrophilic substance to the base material as described in JP 2011 - 110 392 A, and a technique that improves the adhesion while simplifying the structure of the hydrophilic coating film has been sought. Furthermore, for example, although elongated medical instruments that are used by being inserted into a living body include, in addition to those made of metal, those in which the surface is made of urethane, but when a coating film is formed on such elongated medical instruments using an epoxy resin, it can be relatively difficult to obtain sufficient adhesion to the coating film. Therefore, in elongated medical devices provided with a hydrophilic coating film, a technique that suppresses the inconveniences above and enhances the adhesion between a hydrophilic coating film and a base material has been sought.

### SOLUTION TO PROBLEM

This object is solved by the subject matter of the independent claim. Further aspects are disclosed in the dependent claims. The present disclosure can be implemented as the aspects described below.
(1) According to an aspect of the present disclosure, an elongated medical device is provided. The elongated medical device has a coating film formed on a surface of a base material, wherein the coating film is composed of a polymeric material in which a copolymer containing a polymerization unit having a hydrophilic structure has been crosslinked by a structure given by any of formulas (1) to (3).

   (1): -CH(OH)-CH(R¹)-O-C(=O)-NH-R²-NH-C(=O)-O-CH(R¹)-CH(OH)-

   (2): -CH(OH)-CH(R¹)-O-C(=O)-NH-R²-NH-C(=O)-O-CH(CH(R¹)-OH)-

   (3): -CH(CH(R¹)-OH)-O-C(=O)-NH-R²-NH-C(=O)-O-CH(CH(R¹)-OH)-

   (wherein R¹ may be the same or different and represents a hydrogen atom, a linear alkyl group having 1 or more carbon atoms, or a branched alkyl group having 1 or more carbon atoms; and R² is an alkylene group having 1 or more carbon atoms, a divalent alicyclic hydrocarbon group containing an alicyclic structure having 3 or more carbon atoms, or a divalent aromatic group containing an aromatic ring structure having 6 or more carbon atoms, and the alkylene group, the alicyclic hydrocarbon group, and the aromatic group may have a divalent group between the carbon atoms represented by - NR³- (where R³ is a hydrogen atom or an alkyl group having 1 to 8 carbon atoms)).
   According to an elongated medical device of the present aspect, in the coating film formed on the surface of the elongated medical device, it is possible to enhance the adhesion to the base material, and the hydrophilicity.
(2) In the elongated medical device of the aspect above, the hydrophilic structure may be charge neutral. With such a configuration, the biocompatibility of the coating film of the elongated medical device can be enhanced.
(3) In the elongated medical device of the aspect above, the hydrophilic structure may contain at least one type of structure selected from a group consisting of a betaine structure, an amide structure, a lactam structure, and a polyalkylene oxide structure. With such a configuration, an elongated medical device can be provided with a hydrophilic coating film that is charge neutral.
(4) In the elongated medical device of the aspect above, the hydrophilic structure may contain, as a positively charged functional group, at least one of a betaine structure provided with a quaternary ammonium, and an amide structure provided with a tertiary ammonium. With such a configuration, when a coating film is formed on a base material using a coating agent including a copolymer containing a polymerization unit having the hydrophilic structure described above, the tertiary ammonium or quaternary ammonium is capable of acting as a catalyst of a reaction pertaining to the crosslinking of the coating agent. Therefore, it is possible to suppress the addition of another catalyst at the time of forming the coating film and form a hydrophilic coating film using a simpler method, and the composition of the coating film can be simplified.
(5) In the elongated medical device of the aspect above, the base material may contain, on a surface of the base material, at least one of a metal, a polymeric material having a group capable of forming a hydrogen bond, and polyurethane. With such a configuration, the adhesion between the base material and the coating film can be further enhanced.
(6) In the elongated medical device of the aspect above, the elongated medical device may be a guide wire or a catheter. With such a configuration, it is possible to provide a guide wire or a catheter that is provided with a coating film having a strong adhesion to the base material, and a sufficient hydrophilicity.

### BRIEF DESCRIPTION OF DRAWINGS

Fig. 1 is an explanatory diagram representing the crosslinking of a coating agent.
Fig. 2 is an explanatory diagram showing the principle of adhesion of a hydrophilic coating film on a metal base material.
Fig. 3 is an explanatory diagram showing the principle of adhesion of a hydrophilic coating film on a urethane base material.
Fig. 4 is an explanatory diagram showing the results of a cross-cut test.
Fig. 5 is an explanatory diagram showing adhesion evaluation results.
Fig. 6 is an explanatory diagram showing the change in viscosity of coating agents over time.

### EMBODIMENTS OF THE INVENTION

In an elongated medical device of the present embodiment, a hydrophilic coating film is formed on the surface of a base material. In the following description, the coating film provided in the elongated medical device of the present embodiment will firstly be described.

The coating film provided in the elongated medical device of the present embodiment is composed of a polymeric material in which a copolymer containing a polymerization unit having a hydrophilic structure has been crosslinked by a structure given by any of formulas (1) to (3).

(1): -CH(OH)-CH(R¹)-O-C(=O)-NH-R²-NH-C(=O)-O-CH(R¹)-CH(OH)-

(2): -CH(OH)-CH(R¹)-O-C(=O)-NH-R²-NH-C(=O)-O-CH(CH(R¹)-OH)-

(3): -CH(CH(R¹)-OH)-O-C(=O)-NH-R²-NH-C(=O)-O-CH(CH(R¹)-OH)-

Here, R¹ may be the same or different and represents a hydrogen atom, a linear alkyl group having 1 or more carbon atoms, or a branched alkyl group having 1 or more carbon atoms. More specifically, R¹ in each of the above formulas (1) to (3) may independently be a hydrogen atom or an alkyl group having 1 or more carbon atoms. Although the number of carbon atoms in the alkyl group is not limited, for example, it is preferably 1 to 4. R² is an alkylene group having 1 or more carbon atoms, a divalent alicyclic hydrocarbon group containing an alicyclic structure having 3 or more carbon atoms, or a divalent aromatic group containing an aromatic ring structure having 6 or more carbon atoms, and the alkylene group, the alicyclic hydrocarbon group, and the aromatic group may have a divalent group between the carbon atoms represented by - NR³- (where R³ is a hydrogen atom or an alkyl group having 1 to 8 carbon atoms). The number of carbon atoms in the alkylene group is preferably 1 to 5. The number of carbon atoms in the alicyclic structure is preferably 3 to 6, and the number of carbon atoms in the alicyclic hydrocarbon group is preferably 3 to 12. The number of carbon atoms in the aromatic ring structure is preferably 6 to 10, and the number of carbon atoms in the aromatic group is preferably 6 to 20. It is preferable that the number of - NR³- groups that may be present between the carbon atoms of each of the alkylene group, the alicyclic hydrocarbon group, and the aromatic group is 1 or 2. R² is preferably an alkylene group having 1 to 6 carbon atoms, and is particularly preferably an alkylene group having 4 to 6 carbon atoms.

Although the thickness of the coating film is not particularly limited and may be appropriately set according to the application, for example, it is set to approximately 1 µm to 1,000 µm. The coating film can, for example, be formed by a hydrophilic coating agent. In the following description, a preferable coating agent for the formation of a coating film provided in the elongated medical device of the present embodiment will be described.

A preferable coating agent for the formation of the coating film provided in the elongated medical device of the present embodiment includes a copolymer (C) containing a polymerization unit (A) having a cyclic carbonate structure, and a polymerization unit (B) having a hydrophilic structure, as well a solvent. That is, the coating agent used for the formation of the coating film provided in the elongated medical device of the present embodiment includes a copolymer (C) prepared by copolymerizing a material containing a monomer (a) for obtaining the polymerization unit (A) having a cyclic carbonate structure, and a monomer (b) for obtaining the polymerization unit (B) having a hydrophilic structure. In the following description, each of the polymerization units mentioned above will be described.

### (1) Polymerization unit (A) having cyclic carbonate structure:

The polymerization unit (A) having a cyclic carbonate structure described above has at least one of at least one type of cyclic carbonate group. The polymerization unit (A) having a cyclic carbonate structure can have, for example, 1 to 3 cyclic carbonate groups, preferably has 1 or 2 cyclic carbonate groups, and more preferably has 1 cyclic carbonate group.

As the "cyclic carbonate group", the structure represented by the formula (4) below can be used.
In formula (4), R¹ represents any one of a hydrogen atom, a linear alkyl group having 1 to 4 carbon atoms, a branched alkyl group having 1 to 4 carbon atoms, a linear alkenyl group having 1 to 4 carbon atoms, and a branched alkenyl group having 1 to 4 carbon atoms. Here, at least one of the hydrogen atoms of R¹ may be substituted with a halogen atom, and at least one of the carbon atoms (-C-) may be substituted with -O-, -S-, or -P-. Examples of the linear or branched alkyl group having 1 to 4 carbon atoms in R¹ include a methyl group, an ethyl group, an n-propyl group, an isopropyl group, an n-butyl group, and an isobutyl group. Examples of the linear or branched alkenyl group having 1 to 4 carbon atoms include groups in which at least one, and preferably one, of the direct carbon-carbon bonds of the alkyl groups mentioned above is replaced by an unsaturated double bond. From the viewpoint of easily improving the water resistance, R¹ is preferably a hydrogen atom or a methyl group, and is more preferably a hydrogen atom.
In formula (4), R² represents a linear or branched alkylene group or alkenylene group having 1 to 4 carbon atoms. Here, at least one of the hydrogen atoms of R² may be substituted with a halogen atom, and at least one of the carbon atoms (-C-) may be substituted with -O-, -S-, or -P-. Examples of the linear or branched alkylene group having 1 to 4 carbon atoms in R² include a methylene group, an ethylene group, an n-propylene group, an isopropylene group, an n-butylene group, a methylmethylene group, a methylethylene group, a dimethylethylene group, and a methylpropylene group. Examples of the linear or branched alkenylene group having 1 to 4 carbon atoms include groups in which at least one, and preferably one, of the direct carbon-carbon bonds of the alkylene groups mentioned above is replaced by an unsaturated double bond. From the viewpoint of easily improving the water resistance, R² is preferably a linear or branched alkylene group having 1 to 4 carbon atoms, and is more preferably a linear alkylene group having 1 carbon atom.
Specifically, a 2-oxo-1,3-dioxolane structure is preferable. More specifically, the "cyclic carbonate group" is preferably a (2-oxo-1,3-dioxolane-4-yl) group.

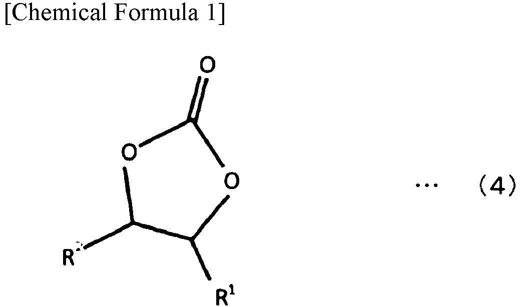

The monomer (a) for forming the polymerization unit (A) having a cyclic carbonate structure is preferably a (meth)acrylate having a cyclic carbonate group, and is more preferably a monomer in which the group represented by formula (5) below is directly bonded to R² of the cyclic carbonate group of formula (4) above.

CH₂=CR¹-R⁴-(CH₂)ₙ ··· (5)

(wherein R¹ represents a hydrogen atom or a methyl group, R⁴ represents -COO- or - CO-NH-, and n represents an integer from 1 to 4).

Specific examples of the monomer (a) include (2-oxo-1,3-dioxolan-4-yl)methyl methacrylate (GCMA) and (2-oxo-1,3-dioxolan-4-yl)methyl acrylate (GCA), and (2-oxo-1,3-dioxolan-4-yl)methyl methacrylate (GCMA) is more preferable.

### (2) Polymerization unit (B) having hydrophilic structure:

The polymerization unit (B) having hydrophilicity in the present embodiment has a hydrophilic structure that imparts hydrophilicity to the polymerization unit. The hydrophilic structure is preferably a charge neutral structure. Examples of the charge neutral hydrophilic structure include a betaine structure, an amide structure, an alkylene oxide structure, and a lactam structure. However, a polymerization unit (B) having a hydrophilic structure other than a betaine structure, an amide structure, an alkylene oxide structure, and a lactam structure as mentioned above may be used, and for example, it is possible to use a polymerization unit having a charged hydrophilic structure that is not charge neutral.

The betaine structure refers to a structure that is neutral (does not have a charge) overall, which has a positive and negative charge at non-adjacent positions within the same molecule, and does not have a dissociable hydrogen bonded to the positively charged atom. In the betaine structure, for example, any of a quaternary ammonium, a sulfonium, and a phosphonium can be used as a functional group having a positive charge, and any one of sulfonate, carboxylate, and phosphonate can be used as a functional group having a negative charge. That is, the betaine structure can be, for example, a sulfobetaine, a carboxybetaine, or a phosphobetaine.

The betaine structure of the present embodiment can have various combinations of the functional group having a positive charge and the functional group having a negative charge as described above. Examples of the betaine structure of the present embodiment that can be preferably used include structures derived from any one of N-methacryloylaminopropyl-N,N-dimethylammonium-α-N-methylcarboxybetaine (MAMCMB), N-methacryloyloxyethyl-N,N-dimethylammonium-α-N-methylcarboxybetaine (CMB), 2-methacryloyl-oxyethyl-phosphorylcholine (MPC), or 3-methacryloylamino-propyl-dimethyl-3-sulfobetaine (SMB). The coating agent used in the formation of the coating film provided in the elongated medical device of the present invention is, as described below, cured by ring-opening the cyclic carbonate structure included in the polymerization unit (A) followed by crosslinking with a crosslinking agent, but when the functional group having a positive charge in the betaine structure is a quaternary ammonium, this is preferable because the quaternary ammonium can act as a catalyst of the reaction pertaining to the crosslinking mentioned above. As an example of the polymerization unit (B) having a betaine structure as the hydrophilic structure, the polymerization unit obtained when N-methacryloylaminopropyl-N,N-dimethylammonium-α-N-methylcarboxybetaine (MAMCMB) is used as the monomer (b) is shown in formula (6) below.

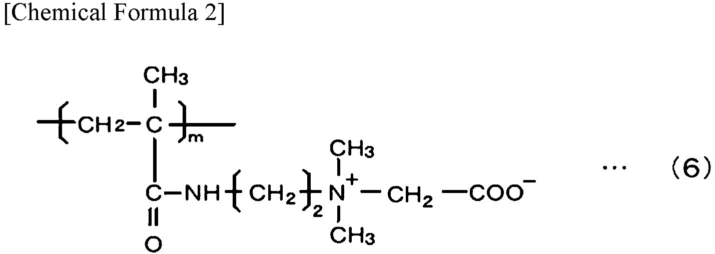

The amide structure is a structure having an amide bond, and is a structure obtained, for example, when any one of N,N-dimethylacrylamide (DMAAm), N-isopropylacrylamide (NiPPAM), acrylamide (Aam), methylacrylamide (MAAm), 2-acrylamido-2-methylpropylsulfonic acid (AMPS), methacrylamide, N-vinylformamide, N-vinylacetamide, and N-vinylpyrrolidone is used as the monomer (b). Among the monomers (b) above, N,N-dimethylacrylamide (DMAAm), N-isopropylacrylamide (NiPPAM), acrylamide (Aam), methylacrylamide (MAAm), and 2-acrylamido-2-methylpropylsulfonic acid (AMPS) can be preferably used as the monomer (b). Such an amide structure has no charge bias and is neutral overall. The coating agent used in the formation of the coating film provided in the elongated medical device of the present invention is, as described below, cured by ring-opening the cyclic carbonate structure included in the polymerization unit (A) followed by crosslinking with a crosslinking agent, but a tertiary ammonium having an amide structure is preferable because it can act as a catalyst of the reaction pertaining to the crosslinking mentioned above. As an example of the polymerization unit (B) having an amide structure as the hydrophilic structure, the polymerization unit obtained when N,N-dimethylacrylamide (DMAAm) is used as a monomer is shown in formula (7) below.

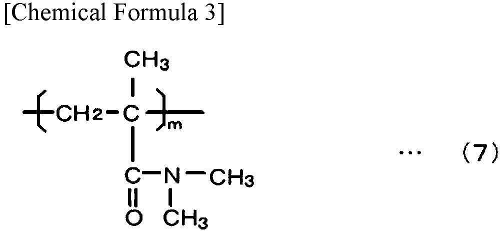

The alkylene oxide structure is a structure having an alkylene oxide group (-RO-; where R is an alkylene group, and R preferably has 1 to 5 carbon atoms). The alkylene oxide structure of the present embodiment is, for example, a structure obtained when any one of alkoxy polyalkylene glycol acrylate, alkoxy polyalkylene glycol methacrylate, alkoxy alkyl acrylate, and alkoxy alkyl methacrylate is used as the monomer (b). Specifically, for example, methoxypolyethylene glycol acrylate, methoxypolyethylene glycol methacrylate, methoxyethyl acrylate, methoxyethyl methacrylate, methoxypolypropylene glycol acrylate, methoxypolypropylene glycol methacrylate, methoxymethyl acrylate, methoxymethyl methacrylate, ethoxymethyl acrylate, ethoxymethyl methacrylate, ethoxyethyl acrylate, ethoxyethyl methacrylate, ethoxypropyl acrylate, and ethoxypropyl methacrylate can be used. Such an alkylene oxide structure has no charge bias and is neutral overall. As an example of the polymerization unit (B) having an alkylene oxide structure as the hydrophilic structure, the polymerization unit obtained when methoxypolyethylene glycol methacrylate (M90G) is used as the monomer (b) is shown in formula (8) below. Furthermore, as another example of the polymerization unit (B) having an alkylene oxide structure as the hydrophilic structure, the polymerization unit obtained when methoxyethyl acrylate (MEA) is used as the monomer (b) is shown in formula (9) below.

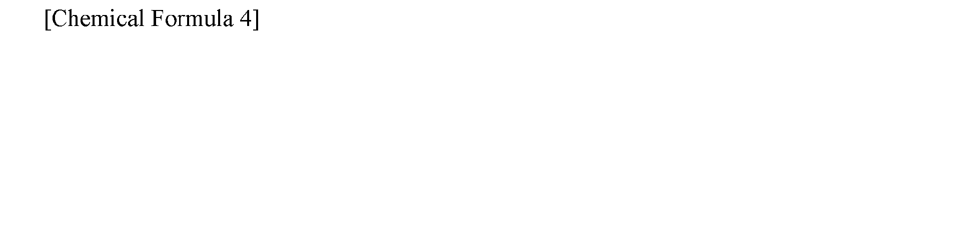

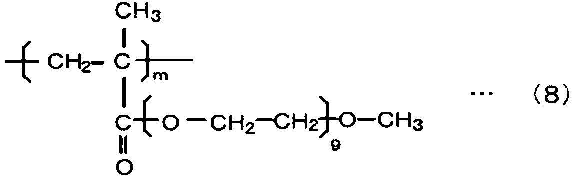

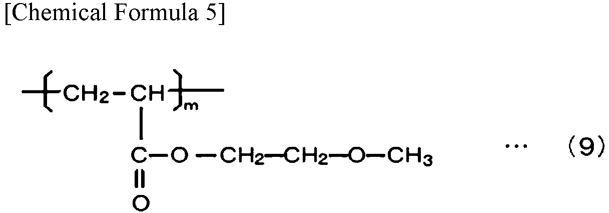

The lactam structure can be a β-lactam (4-membered ring) structure, a γ-lactam (5-membered ring) structure, a δ-lactam (6-membered ring) structure, or an ε-lactam (7-membered ring) structure, and is preferably a γ-lactam (5-membered ring) structure. Examples of the monomer (b) used for obtaining the lactam structure of the present embodiment include vinyl monomers having a 5-membered ring lactam structure such as N-vinylpyrrolidone, N-vinyl-5-methylpyrrolidone, N-vinyl-5-ethylpyrrolidone, N-vinyl-5-propylpyrrolidone, N-vinyl-5-butylpyrrolidone, and 1-(2-propenyl)-2-pyrrolidone, vinyl monomers having a 6-membered ring lactam structure such as N-vinylpiperidone, and vinyl monomers having a 7-membered ring lactam structure such as N-vinylcaprolactam. Such a lactam structure has no charge bias and is neutral overall. As an example of the polymerization unit (B) having a lactam structure as the hydrophilic structure, the polymerization unit obtained when N-vinylpyrrolidone (NVP) is used as the monomer (b) is shown in formula (10) below.

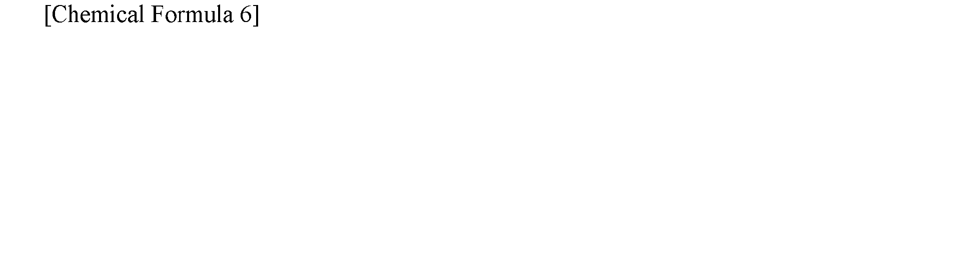

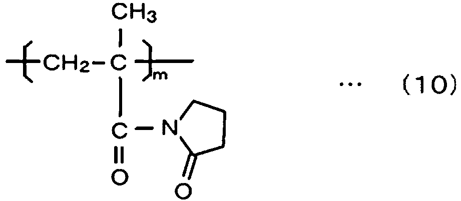

The polymerization unit (B) having hydrophilicity can be formed using various hydrophilic monomers (b) other than those described above. Examples of other usable hydrophilic monomers (b) include acrylic acid and acrylates such as sodium acrylate, methacrylic acid and methacrylates such as sodium methacrylate, maleic anhydride, 2-hydroxyethyl methacrylate (HEMA), 2-hydroxyethyl acrylate (2HEA), 2-hydroxypropyl acrylate (2HPA), 2-hydroxypropylmethyl acrylate (2HPMA), 4-hydroxybutyl acrylate (4HBA), 4-hydroxybutyl methacrylate (4HBMA), 1,4-cyclohexanedimethanol monoacrylate (CHDMA), lactic acid and other amino acids, acryloylmorpholine (AMP), and N,N-dimethylaminoethyl acrylate.

### (3) Copolymer (C):

As mentioned above, the coating agent used for the formation of the coating film provided in the elongated medical device of the present embodiment includes a copolymer (C) containing a polymerization unit (A) having a cyclic carbonate structure, and a polymerization unit (B) having a hydrophilic structure. As mentioned above, the copolymer (C) included in the coating agent can be prepared by copolymerizing a material containing a monomer (a) for obtaining the polymerization unit (A) having a cyclic carbonate structure, and a monomer (b) for obtaining the polymerization unit (B) having a hydrophilic structure.

The copolymer (C) may have, as the polymerization unit (A) having a cyclic carbonate structure, a structural unit derived from one or more types of monomers (a) selected from the monomers (a) mentioned above. Furthermore, the copolymer (C) may have, as the polymerization unit (B) having a hydrophilic structure, a structural unit derived from one or more types of monomers (b) selected from the monomers (b) mentioned above. Moreover, the copolymer (C) may be a random copolymer containing the polymerization unit (A) having a cyclic carbonate structure and the polymerization unit (B) having a hydrophilic structure, a block copolymer, or a mixture thereof.

The copolymer (C) may further contain a structural unit that is different from the polymerization unit (A) having a cyclic carbonate structure and the polymerization unit (B) having a hydrophilic structure. For example, at the time of production of the copolymer (C), a monomer having a long-chain aliphatic structure such as n-butyl methacrylate or n-lauryl methacrylate may be added in addition to the monomer (a) and the monomer (b). As a result, the glass transition point Tg of the copolymer (C) can be lowered to soften the copolymer (C). Also, at the time of production of the copolymer (C), a monomer having a functional group capable of forming crosslinks upon irradiation with light, such as 4-methacryloyloxybenzophenone (MBP) or 4-methacryloyloxy-2-hydroxybenzophenone (MHP), may be added in addition to the monomer (a) and the monomer (b).

In the copolymer (C) described above, from the viewpoint of ensuring adhesion between the coating agent and the base material to be coated with the coating agent, the content of the polymerization unit (A) having a cyclic carbonate structure is preferably 2 mol% or more, more preferably 3 mol % or more, and even more preferably 5 mol % or more. Furthermore, the content of the polymerization unit (A) having a cyclic carbonate structure may be, for example, 50 mol% or less, and is preferably 30 mol% or less, more preferably 20 mol% or less, and even more preferably 15 mol% or less. In the copolymer (C), from the viewpoint of ensuring the hydrophilicity of the coating film, the content of the polymerization unit (B) having a hydrophilic structure may be, for example, 50 mol% or more, and from the viewpoint of providing an elongated medical device having excellent lubricity, is preferably 70 mol% or more, more preferably 80 mol% or more, and even more preferably 85 mol% or more. Moreover, in the copolymer (C), the content of the polymerization unit (B) having a hydrophilic structure is preferably 98 mol% or less, more preferably 97 mol% or less, and even more preferably 95 mol% or less. When producing the copolymer (C), for example, the monomer (a) is mixed at the ratio of the polymerization unit (A) having a cyclic carbonate structure mentioned above, and the monomer (b) is mixed at the ratio of the polymerization unit (B) having a hydrophilic structure mentioned above.

The copolymer (C) described above preferably has at least a polymerization unit (B 1) having a betaine structure as the polymerization unit (B) having a hydrophilic structure, and from the viewpoint of easily enhancing the lubricity of the coating film, the amount of the polymerization unit (B 1) having a betaine structure is preferably 10 mol% or more, more preferably 20 mol% or more, even more preferably 30% or more, and still more preferably 40% or more based on the total amount of the polymerization units contained in the copolymer (C). The copolymer (C) containing a polymerization unit having a betaine structure preferably also includes, as the polymerized unit (B) having a hydrophilic structure, at least one type of structure selected from a group consisting of an amide structure, an alkylene oxide structure, and a lactam structure.

In addition, the copolymer (C) described above preferably has at least a polymerization unit (B2) having an amide structure as the polymerization unit (B) having a hydrophilic structure, and from the viewpoint of easily enhancing the lubricity of the coating film and easily increasing the crosslinking of the coating film, the amount of the polymerization unit (B2) having an amide structure may be 10 mol% or more, and is preferably 30 mol% or more, more preferably 50 mol% or more, even more preferably 70 mol% or more, still more preferably 80 mol% or more, and particularly preferably 85 mol% or more based on the total amount of the polymerization units contained in the copolymer (C). Also, the copolymer (C) containing a polymerization unit having an amide structure preferably also includes, as the polymerized unit (B) having a hydrophilic structure, at least one type of structure selected from a group consisting of a betaine structure, an alkylene oxide structure, and a lactam structure.

The weight average molecular weight of the copolymer (C) is preferably 10,000 or more, and more preferably 40,000 or more. Furthermore, the weight average molecular weight of the copolymer (C) is preferably 1,000,000 or less, and more preferably 90,000 or less.

The method of polymerizing the material containing the monomer (a) and the monomer (b) when producing the copolymer (C) is not particularly limited, and examples include a solution polymerization method, a bulk polymerization method, an emulsion polymerization method, and a suspension polymerization method. Among these, a solution radical polymerization method is preferred.

### (4) Formation of hydrophilic coating film:

The hydrophilic coating film provided in the elongated medical device of the present embodiment can be formed by coating a base material using a coating agent containing the copolymer (C) described above, and a solvent. Specifically, the coating agent described above is further mixed with a crosslinking agent such as a diamine or a polyamine, and polyhydroxyurethane is formed by reacting the cyclic carbonate structure of the polymerization unit (A) with a crosslinking agent to ring-open the cyclic carbonate. By allowing such a reaction to proceed on a base material coated with the coating agent, a hydrophilic coating film that adheres to the base material can be formed.

The crosslinking agent used for forming the polyhydroxyurethane is not particularly limited as long as it is a material having two or more primary amines in the molecule, and amine crosslinking agents such as an aliphatic polyamine, an alicyclic polyamine, or an aromatic polyamine can be preferably used. Specifically, as the aliphatic polyamine, for example, hexamethylenediamine (HMDA), 1,4-butanediamine (BDA), diethylenetriamine (DETA), and triethylenetetramine (TETA) can be preferably used. As the alicyclic polyamine, for example, mensenediamine (MDA) and isophoronediamine (IPDA) can be preferably used. As the aromatic polyamine, for example, metaxylene diamine (m-XDA), diaminodiphenylmethane (DDM), and m-phenylene diamine (m-PDA) can be preferably used. Specifically, hexamethylene diamine (HMDA) is a long-chain aliphatic compound, has high structural reactivity and flexibility, and is suitable as a crosslinking agent. Furthermore, it also has lower toxicity than other diamine compounds with shorter chain lengths, making it suitable for medical device applications.

When applying the coating agent onto the base material, the coating agent and the crosslinking agent may be dissolved in a solvent so that the concentration and viscosity of the coating agent fall within an appropriate range. The amount of the solvent is not particularly limited as long as it is an amount capable of dissolving the copolymer (C) and the cross-linking agent, and may be appropriately adjusted to an amount that is easy to coat, and for example, may be 10 to 99% by mass of the total coating agent that is obtained. The solvent is not particularly limited as long as it is capable of dissolving the copolymer (C). For example, alcohols such as ethanol, methanol, propanol, 2-propanol, butanol, and benzyl alcohol, and various hydrophilic polar solvents such as N-methyl-2-pyrrolidone (NMP), dimethyl sulfoxide (DMSO), N,N-dimethylformamide (DMF), and dimethylacetamide (DMA) can be used. In the coating agent, a polymerization initiator or catalyst may be added as necessary. The coating agent mixed with the crosslinking agent can be cured by heating to, for example, 70 to 150°C so as to cause the ring-opening reaction of the cyclic carbonate described above to proceed.

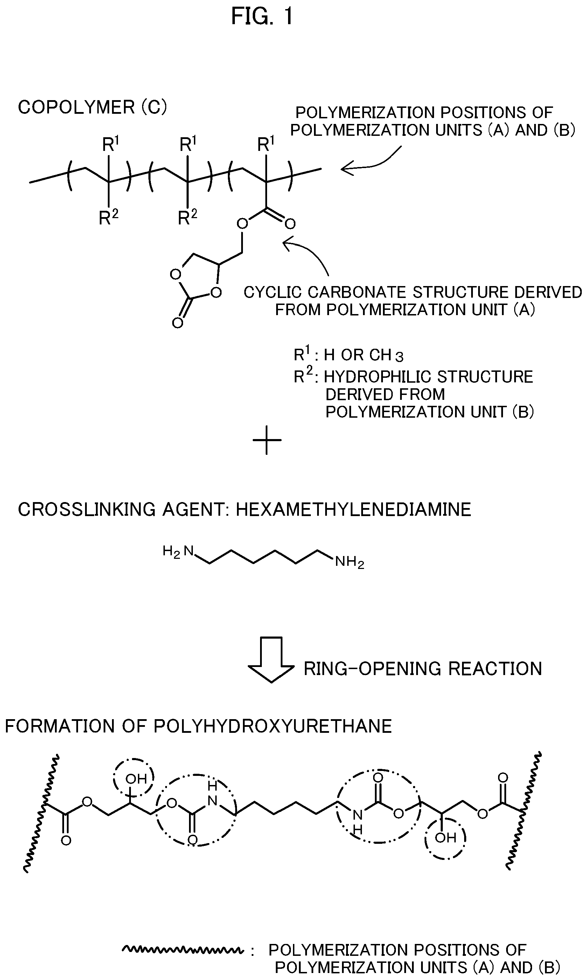

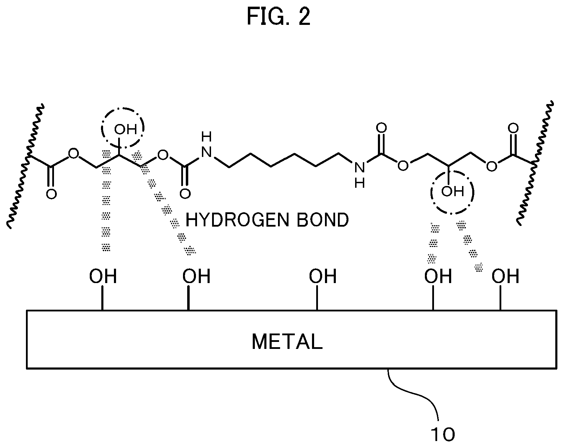

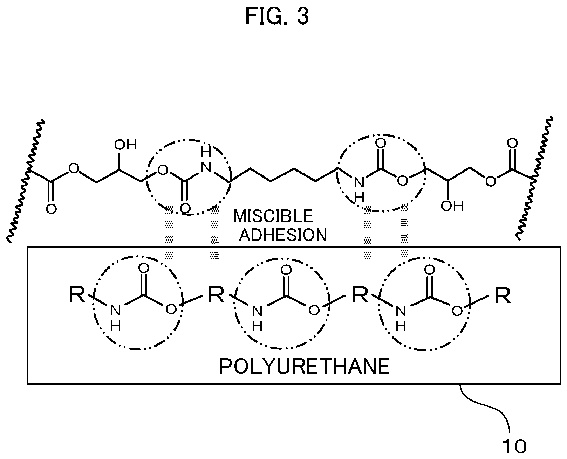

Fig. 1 is an explanatory diagram representing the crosslinking of the copolymer (C) contained in the coating agent by a crosslinking agent to form polyhydroxyurethane. In Fig. 1, the cyclic carbonate structure is shown for a case where, as the cyclic carbonate structure derived from the polymerization unit (A) included in the copolymer (C), (2-oxo-1,3-dioxolan-4-yl)methyl methacrylate (GCMA) is used as the monomer (a). In the copolymer (C) shown in Fig. 1, R¹ is a hydrogen atom (H) or a methyl group (CH₃), but when (2-oxo-1,3-dioxolan-4-yl)methyl methacrylate (GCMA) is used as the monomer (a), R¹ is H. Furthermore, Fig. 1 shows the use of hexamethylenediamine as the crosslinking agent. Note that, in the section of Fig. 1 that represents the state after the ring-opening reaction, and in Figs. 2 and 3 described below, the "portion representing the polymerization positions of the polymerization units (A) and (B)" is simplified and represented by wavy lines, and the "hydrophilic structure R² derived from the polymerization unit (B)" is omitted.

As shown in Fig. 1, when the coating agent is cured, a urethane bond is formed and a hydroxyl group is generated with the ring-opening of the cyclic carbonate. In Fig. 1, the hydroxyl groups are enclosed by a one-dot chain line, and the urethane bonds are enclosed by a two-dot chain line. When the cyclic carbonate is ring-opened, the obtained crosslinked structure may vary depending on the position attacked by the amine of the crosslinking agent. That is, as shown in Fig. 1, when ring-opening of a structure derived from (2-oxo-1,3-dioxolan-4-yl)methyl methacrylate (GCMA) occurs, the three types of crosslinked structures shown in formulas (1) to (3) below can be generated depending on which of the oxygen atoms in position numbers 1 and 3 of the five-membered ring is attacked by the amine of the crosslinking agent. Fig. 1 shows a case where the crosslinked structure of formula (1) is obtained.

(1): -CH(OH)-CH(R¹)-O-C(=O)-NH-R²-NH-C(=O)-O-CH(R¹)-CH(OH)-

(2): -CH(OH)-CH(R¹)-O-C(=O)-NH-R²-NH-C(=O)-O-CH(CH(R¹)-OH)-

(3): -CH(CH(R¹)-OH)-O-C(=O)-NH-R²-NH-C(=O)-O-CH(CH(R¹)-OH)-

(wherein R¹ may be the same or different and represents a hydrogen atom, a linear alkyl group having 1 or more carbon atoms, or a branched alkyl group having 1 or more carbon atoms; and R² is an alkylene group having 1 or more carbon atoms, a divalent alicyclic hydrocarbon group containing an alicyclic structure having 3 or more carbon atoms, or a divalent aromatic group containing an aromatic ring structure having 6 or more carbon atoms, and the alkylene group, the alicyclic hydrocarbon group, and the aromatic group may have a divalent group between the carbon atoms represented by - NR³- (where R³ is a hydrogen atom or an alkyl group having 1 to 8 carbon atoms)).

In the present embodiment, the material forming the base material to be coated with the coating agent is not particularly limited, and may be, for example, a metal or a polymeric material (resin). From the viewpoint of further enhancing the adhesion of the coating agent, the surface of the base material preferably contains at least one of a metal, a polymeric material having a group capable of forming a hydrogen bond, and polyurethane. For example, by coating and curing the coating agent described above on a metal base material, a base material containing a polymeric material having a group capable of forming a hydrogen bond, or a polyurethane base material, the adhesion between the obtained hydrophilic coating film (hydrogel layer) and the base material can be further enhanced.

As the metal forming the metal base material, for example, it is possible to use an element that forms metallic bonds such as iron (Fe), chromium (Cr), nickel (Ni), molybdenum (Mo), cobalt (Co), titanium (Ti), tungsten (W), platinum (Pt), gold (Au), silver (Ag), and tin (Sn), which can be used alone or in the form of an alloy. More specifically, a stainless alloy, a nickel-titanium alloy, a cobalt-chromium alloy, a platinum alloy, tungsten, a tin-silver alloy, and the like can be preferably used.

The "group capable of forming a hydrogen bond" mentioned above contains a hydrogen atom, and is also a group in which a covalent bond is formed between the hydrogen atom and an atom having a higher electronegativity than the hydrogen atom. Examples of atoms that form a covalent bond with a hydrogen atom include an oxygen atom (O), a nitrogen atom (N), a sulfur atom (S), and a carbon atom (C). More specifically, examples of the "group capable of forming a hydrogen bond" include a hydroxyl group (-OH), an amino group (-NH₂), and an imino group (=NH). Furthermore, as the "polymeric material having a group capable of forming a hydrogen bond", for example, polyvinyl alcohol (PVA), a modified polyolefin resin having a group capable of forming a hydrogen bond, and the like, can be used.

As the polyurethane base material, a wide variety of synthetic resins having a urethane bond can be used. Specifically, for example, aromatic ether urethane, aromatic carbonate urethane, aromatic ester urethane, aliphatic ether urethane, aliphatic carbonate urethane, aliphatic ester urethane, polyhydroxy urethane, urea urethane having a portion with a urea bond, and the like, can be used. In particular, aromatic ether urethane and polyhydroxy urethane are preferably used because they have superior flexibility, reactivity, and adhesion.

As an example of the formation of a hydrophilic coating film, the formation of a hydrophilic coating film using a base material whose surface is formed of metal, a base material having a polymeric material having a group capable of forming a hydrogen bond on the surface, and a base material whose surface is formed of polyurethane will be described below.

Fig. 2 is an explanatory diagram schematically showing the principle of adhesion of the hydrophilic coating film according to the present embodiment on a base material 10 whose surface is formed of metal. As shown in Fig. 2, when the coating agent of the present embodiment is cured, a hydroxyl group is generated with the ring-opening of the cyclic carbonate. The hydroxyl group generated in this manner forms a hydrogen bond with a hydroxyl group on the base material surface formed of metal, and adheres to the base material surface. That is, a reaction that ring-opens and crosslinks the cyclic carbonate and cures the coating agent, and a reaction that causes a hydrogen bond to be formed with the base material surface as mentioned above proceed simultaneously, and the hydrophilic coating film adheres to the base material 10. By the same principle, the hydrophilic coating film is also firmly adhered to the base material 10 provided with a polymeric material having a group capable of forming a hydrogen bond on the surface.

Fig. 3 is an explanatory diagram schematically showing the principle of adhesion of the hydrophilic coating film according to the present embodiment on a base material 10 whose surface is formed of polyurethane. As shown in Fig. 3, when the coating agent of the present embodiment is cured, a urethane bond is generated with the ring-opening of the cyclic carbonate. The urethane bond generated in this manner miscibly adheres to the urethane forming the base material surface. That is, a reaction that ring-opens and crosslinks the cyclic carbonate and cures the coating agent, and a reaction that causes the urethanes to miscibly adhere to those on the base material surface as mentioned above proceed simultaneously, and the hydrophilic coating film adheres to the base material 10.

However, as the base material provided in the elongated medical device of the present embodiment, a base material that is different from a base material including at least one of a metal, a polymeric material having a group capable of forming a hydrogen bond, or polyurethane on the surface may be used. For example, when the coating agent is cured on the base material to form a coating film, the same strong adhesion can be obtained in the hydrophilic coating film as long as a bonding force is generated due to hydrogen bonding between the coating film and the base material.

### (5) Elongated medical device:

An elongated medical device of the present embodiment can be used as a medical device inserted into a living body. An elongated medical device of the present embodiment can be used as a medical device inserted into a living body. Specifically, in an elongated medical device of the present embodiment, for example, an elongated medical device made of metal, an elongated medical device made of urethane, or an elongated medical device in which a metal is coated by polyurethane or a polymeric material having a group capable of forming a hydrogen bond is used as the base material 10, and an elongated medical device can be obtained in which the hydrophilic coating film described above is formed on the surface of the base material 10. Particularly preferable examples of the elongated medical device of the present embodiment include a guide wire and a catheter. Specifically, for example, a guide wire made of metal, a guide wire provided with a urethane coating layer on the surface of a coil layer on a distal end portion (urethane jacket guide wire), a catheter provided with a hollow shaft made of polyurethane, and the like, can be used as the base material.

The catheter of the present disclosure is not particularly limited, and can be applied, for example, to any type of catheter such as a guiding catheter, a penetrating catheter, a microcatheter, a balloon catheter, a foreign body removal catheter, a contrast imaging catheter, a bile duct catheter, a urethral catheter, an endoscope, or a dilator. Furthermore, the guide wire of the present disclosure is not particularly limited, and can be applied, for example, to any type of guide wire such as a PCI guide wire for coronary artery treatment, a PTA guide wire for lower limb vascular treatment, an IVR guide wire for peripheral vascular treatment, an INR guide wire for cerebrovascular treatment, and a CAG guide wire for contrast imaging.

More specifically, the elongated medical device of the present embodiment can adopt various configurations such as those described in (a) to (e) below. The coating layer provided in the guide wires described in (a) and (c) below, as described in terms of the configuration of the surface of the base material 10 above, preferably includes at least one of a metal, a polymeric material having a group capable of forming a hydrogen bond, and polyurethane.
(a) A guide wire including: a linear core wire; a coating layer provided on at least a portion of an outer periphery of the core wire; and a coating film formed on a surface of the coating layer, the coating film being formed of a polymeric material in which a copolymer (C) containing a polymerization unit (B) having a hydrophilic structure that has been crosslinked by a structure represented by any of formulas (1) to (3) described above.
(b) A guide wire including: a linear core wire; a coil layer in which a wire material is spirally wound around at least a portion of an outer periphery of the core wire; and a coating film formed on a surface of the coil layer, the coating film being formed of a polymeric material in which a copolymer (C) containing a polymerization unit (B) having a hydrophilic structure that has been crosslinked by a structure represented by any of formulas (1) to (3) described above.
(c) A guide wire including: a linear core wire; a coil layer in which a wire material is spirally wound around at least a portion of an outer periphery of the core wire; a coating layer provided on an outer periphery of the coil layer; and a coating film formed on a surface of the coating layer, the coating film being formed of a polymeric material in which a copolymer (C) containing a polymerization unit (B) having a hydrophilic structure that has been crosslinked by a structure represented by any of formulas (1) to (3) described above.
(d) A catheter including: a tubular member; and a coating film formed on a surface of the tubular member, the coating film being formed of a polymeric material in which a copolymer (C) containing a polymerization unit (B) having a hydrophilic structure that has been crosslinked by a structure represented by any of formulas (1) to (3) described above.
(e) A catheter including: a tubular member; a balloon disposed on one end of the tubular member; and a coating film formed on a surface of the balloon, the coating film being formed of a polymeric material in which a copolymer (C) containing a polymerization unit (B) having a hydrophilic structure that has been crosslinked by a structure represented by any of formulas (1) to (3) described above.

However, the elongated medical device of the present embodiment may have a configuration different from (a) to (e) above, and may be an elongated medical device other than a guide wire or a catheter. At least a portion of the surface of the elongated medical device is provided with a hydrophilic coating film formed using the coating agent according to the present embodiment described above.

When the elongated medical device of the present embodiment includes, as the base material, a resin that is difficult to heat, it is preferable to form the hydrophilic coating film on the base material at a lower temperature. Therefore, in such a case, even under relatively low temperature conditions, as the copolymer (C) constituting the coating film, it is preferable to use a copolymer (C) that acts as a catalyst of a reaction that ring-opens and crosslinks the cyclic carbonate structure of the polymerization unit (A). As mentioned above, for example, when the copolymer (C) includes, as the polymerization unit (B) having a hydrophilic structure, a betaine structure provided with a quaternary ammonium or an amide structure provided with a tertiary ammonium, the quaternary ammonium or the tertiary ammonium can act as a catalyst of the reaction pertaining to the crosslinking. In particular, a tertiary ammonium, and specifically a tertiary ammonium that does not form a ring structure, is preferable because it has a high activity with respect to promoting the reaction pertaining to the crosslinking even under low-temperature conditions such as room temperature. From the viewpoint of causing the crosslinking of the copolymer (C) to efficiently proceed at a low temperature, it is preferable that the copolymer (C) is a random copolymer of the polymerization unit (A) and the polymerization unit (B) described above.

The elongated medical device of the present embodiment configured as described above includes a coating film formed by using a coating agent containing the copolymer (C), which includes the polymerization unit (A) having a cyclic carbonate structure, and the polymerization unit (B) having a hydrophilic structure. As a result, in the coating film formed on the surface of the elongated medical device, it is possible to enhance the adhesion to the base material, and the hydrophilicity. Then, by coating the coating agent on the base material, and forming a coating film by performing a curing reaction involving the ring-opening of the cyclic carbonate, for example, it is possible to form a hydrophilic coating film while ensuring adhesion to the base material without separately providing a layer for enhancing the adhesion to the base material.

Because a hydroxyl group is generated with the ring-opening of the cyclic carbonate of the polymerization unit (A) of the coating agent, when a metallic member having hydroxyl groups on the surface is used as the base material, hydrogen bonding occurs between the hydroxyl group generated with the ring-opening of the cyclic carbonate and the hydroxyl groups on the surface of the base material made of metal. As a result, as the hydrophilic coating film is formed, it becomes possible for the hydrophilic coating film to become well adhered to the base material. Furthermore, because a urethane bond is formed with the ring-opening of the cyclic carbonate of the polymerization unit (A) of the coating agent, when the surface of the base material is formed of a urethane resin, the urethane bond that is formed with the ring-opening of the cyclic carbonate and the urethane structure of the base material surface are miscible. As a result, as the hydrophilic coating film is formed, it becomes possible for the hydrophilic coating film to become well adhered to the base material.

In this way, because hydroxyl groups or urethane bonds are generated on the base material surface due to a reaction on the base material surface that proceeds after coating the coating agent on the base material, interactions occur more easily between the hydrophilic coating film and the base material surface, and it becomes easier to obtain strong adhesion. Furthermore, when the coating agent according to the present embodiment is used, a hydrophilic coating film that adheres well to the base material can be formed when the surface of the base material is formed of metal, or formed of a polymeric material having a group capable of forming a hydrogen bond, or formed of a urethane resin, and the coating agent can be broadly used with respect to common metals and polymeric materials including urethane resins that are used as a surface structure of an elongated medical instrument. Therefore, the coating agent according to the present embodiment can be used as a coating agent having higher versatility than a conventionally known hydrophilic coating agent for an elongated medical device.

In particular, when the coating agent is used to coat the elongated medical device, the biocompatibility of the elongated medical device can be enhanced by making the hydrophilic structure of the polymerization unit (B) having a hydrophilic structure a charge neutral structure. In contrast, for example, when the coating agent has a charged structure that is not neutral as the hydrophilic structure, it is possible that the biocompatibility will be insufficient. Specifically, for example, because protein in the blood is attracted to electric charge and is likely to become adsorbed, thrombus formation may be promoted, or allergic reactions may arise due to adsorption of complement proteins. As an example, formula (11) below shows a polymerization unit obtained when methacrylic acid, which has a carboxylic acid having a negatively charged hydrophilic structure, is used as a monomer. In the elongated medical device of the present embodiment, which is coated using the coating agent according to the present embodiment, because the hydrophilic structure of the coating film is charge neutral, such a reaction can be suppressed, and the biocompatibility can be enhanced.

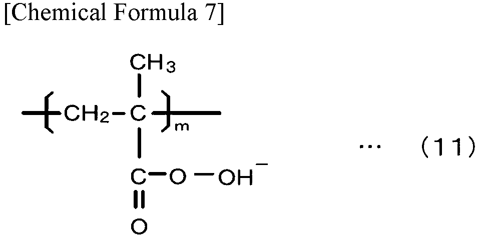

Furthermore, when forming the hydrophilic coating film of the elongated medical device, when a ring-opening reaction of the cyclic carbonate structure of the polymerization unit (A) is used, a urethane reaction can be made to proceed while suppressing the use of a harmful substance such as isocyanate. Therefore, for example, even when the elongated medical device is used as the base material, it is possible to eliminate or simplify the post-treatment performed after the coating process using the coating agent, and the entire production process including the coating process using the coating agent can be simplified. Furthermore, because the ring-opening reaction of the cyclic carbonate structure generally proceeds under relatively mild conditions of approximately 70°C, the production cost of a device provided with the hydrophilic coating film can be suppressed.

### [Examples]

The elongated medical device of the present disclosure will be described below based on examples. Here, the elongated medical devices of samples S1 to S7 were prepared and compared by coating a base material using coating agents obtained using different conditions pertaining to the polymerization unit (B) having a hydrophilic structure, and different conditions pertaining to the curing (gelation).

### <Description of samples>

The copolymer used to prepare the elongated medical device of sample S1 contained a structural unit derived from (2-oxo-1,3-dioxolan-4-yl)methyl methacrylate (GCMA) as the polymerization unit (A) having a cyclic carbonate structure. Furthermore, the copolymer used to prepare sample S1 contained structural units derived from N-methacryloylaminopropyl-N,N-dimethylammonium-α-N-methylcarboxybetaine (MAMCMB) and methoxypolyethylene glycol methacrylate (M90G) as the polymerization unit (B) having a hydrophilic structure. Specifically, the copolymer used to prepare sample S1 contained 10 mol% of the structural unit derived from GCMA, 40 mol% of the structural unit derived from MAMCMB, and 50 mol% of the structural unit derived from M90G. Hereinafter, the copolymer used to prepare sample S1 is also referred to as "Poly(MAMCMB-M90G-GCMA) 40:50:10". "Poly(MAMCMB-M90G-GCMA) 40:50: 10" is represented by formula (12) below. "Poly(MAMCMB-M90G-GCMA) 40:50:10" is a random copolymer, but formula (12) represents a site where the three structural units described above are consecutively polymerized. In formula (12), the cyclic carbonate is shown enclosed by a broken line.

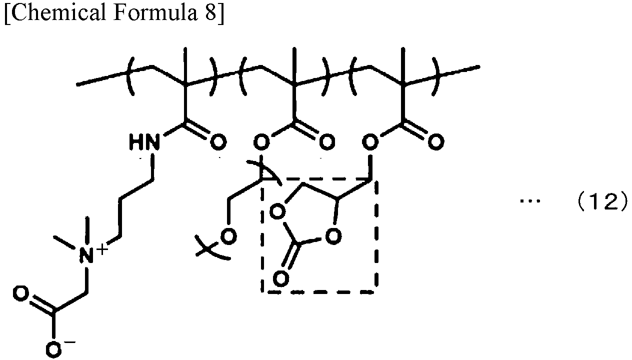

The elongated medical device of sample S1 was prepared by using a medical guide wire provided with a metal coil portion as the base material, and coating a part of the base material including the metal coil portion with "Poly(MAMCMB-M90G-GCMA) 40:50:10". Specifically, the copolymer described above was dissolved in ethanol to a concentration of 20 wt%. Then, immediately before being coated on the base material, the coating agent having the copolymer dissolved therein was mixed with a 5% hexamethylene diamine (HMDA) ethanol solution as a crosslinking agent at a weight ratio of 5:3, and was sufficiently dissolved. The coating agent having the crosslinking agent added thereto was coated on the base material by a dip coating method. After applying the coating agent, sample S1 was prepared by drying for 1 hour using a hot air circulation drying oven at 120°C.

The elongated medical device of sample S2 was prepared in the same manner as sample S1, except for using a guide wire provided with a urethane coating layer on the surface of the metal coil portion as the base material (urethane jacket guide wire), and coating the coating agent on a part including the urethane coating layer.

The copolymer used to prepare the elongated medical device of sample S3 contained a structural unit derived from (2-oxo-1,3-dioxolan-4-yl)methyl methacrylate (GCMA) as the polymerization unit (A) having a cyclic carbonate structure. Furthermore, the copolymer used to prepare sample S3 contained a structural unit derived from N,N-dimethylacrylamide (DMAAm) as the polymerization unit (B) having a hydrophilic structure. Specifically, the copolymer used to prepare sample S3 contained 10 mol% of the structural unit derived from GCMA, and 90 mol% of the structural unit derived from DMAAm. Hereinafter, the copolymer used to prepare sample S3 is also referred to as "Poly(DMAAm -GCMA) 90:10". "Poly(DMAAm - GCMA) 90: 10" is represented by formula (13) below. "Poly(DMAAm -GCMA) 90: 10" is a random copolymer, and formula (13) represents a site where the two structural units described above are consecutively polymerized. In formula (13), the cyclic carbonate is shown enclosed by a broken line, and the tertiary ammonium is shown enclosed by a one-dot chain line. The weight average molecular weight of the copolymer pertaining to sample S3 was approximately 90,000. The weight average molecular weight of the copolymer pertaining to sample S3 and the weight average molecular weight of the copolymers pertaining to the other samples described below were measured by the gel permeation chromatography (GPC) method.

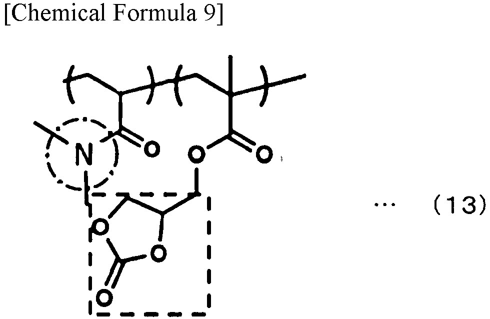

The elongated medical device of sample S3 was prepared in the same manner as sample S1 by using a medical guide wire provided with a metal coil portion as the base material, and coating a part of the base material including the metal coil portion with "Poly(DMAAm -GCMA) 90: 10". Except for changing the solvent used when dissolving the copolymer and preparing the coating agent from ethanol to dimethylformamide, the method of coating the base material with the coating agent when preparing sample S3 was the same as that of sample S1.

The elongated medical device of sample S4 was prepared in the same manner as sample S3, except for using a guide wire provided with a urethane coating layer on the surface of the metal coil portion as the base material (urethane jacket guide wire), and coating the coating agent on a part including the urethane coating layer.

In the same manner as sample S4, the elongated medical device of sample S5 used "Poly(DMAAm -GCMA) 90: 10" as the copolymer, and used a guide wire provided with a urethane coating layer on the surface of the metal coil portion as the base material (urethane jacket guide wire). However, the copolymer used to produce the elongated medical device of sample S5 had a different weight average molecular weight to the copolymer used to produce sample S4 (and sample S3) because the conditions used when preparing the copolymer were different. The weight average molecular weight of the copolymer pertaining to sample S5 was approximately 40,000.

In the same manner as sample S1, the elongated medical device of sample S6 used "Poly(MAMCMB-M90G-GCMA) 40:50:10" as the copolymer, and used a medical guide wire provided with a metal coil portion as the base material. However, in sample S6, a hydrophilic coating film was formed on the base material using the coating agent under the same conditions as in sample S1 but without adding HDMA to the coating agent as a crosslinking agent. Sample S6 corresponds to a comparative example.

In the same manner as sample S3, the elongated medical device of sample S7 used "Poly(DMAAm -GCMA) 90: 10" as the copolymer, and used a medical guide wire provided with a metal coil portion as the base material. However, in sample S7, a hydrophilic coating film was formed on the base material using the coating agent under the same conditions as in sample S1 but without adding HDMA to the coating agent as a crosslinking agent. Sample S7 corresponds to a comparative example.

### <Evaluation of lubricity>

To evaluate the lubricity, each of the prepared samples S1 to S7 was immersed in physiological saline, and then the sensation when the coated section was rubbed between the fingertips was compared. The evaluation results are summarized in Table 1 below. In Table 1, the evaluation results of samples with a good slip evaluation result are indicated with an "A", and the evaluation results of samples with a poor slip evaluation result are indicated with a "B". As shown in Table 1, it was confirmed that samples S1 to S5 had good lubricity unlike samples S6 and S7. That is, it was confirmed that a hydrophilic coating film that adheres to the surface of the base material and exhibits sufficient lubricity was formed as a result of the crosslinking reaction of the coating agent proceeding on the base material. Furthermore, it was confirmed that even when the molecular weight of the copolymer used to form the hydrophilic coating film was different as in sample S4 and sample S5, for example, a hydrophilic coating film exhibiting good lubricity can be formed over a wide range of 40,000 to 90,000 weight average molecular weight.

**[Table 1]**

| | Slip Evaluation | Surface State |
|---|---|---|
| Sample S1 | A | Slimy |
| Sample S2 | A | Slimy |
| Sample S3 | A | Slimy |
| Sample S4 | A | Slimy |
| Sample S5 | A | Slimy |
| Sample S6 | B | Leaking out in water |
| Sample S7 | B | Leaking out in water |

### <Evaluation of film strength>

To evaluate the film strength, each of the prepared samples S1 to S7 was held between a urethane roller (AXFM-D25-L15-V8-N, manufactured by Misumi Co., Ltd.) and a stainless steel plate (SUS304 plate, 30 x 30 mm) in an underwater environment, and the resistance value was measured when one end connected to a load cell was pulled out under a load of 0.981N. The same measurement was consecutively performed 50 times, and the initial resistance value from the first measurement and the resistance value from the 50th measurement were compared to evaluate the film strength. A smaller resistance value can be evaluated as a better film strength. The evaluation results are summarized in Table 2 below. As shown in Table 2, it was confirmed that samples S1 to S5 exhibited a low resistance value even in the 50th measurement unlike samples S6 and S7. That is, it was confirmed that a hydrophilic coating film that adheres to the surface of the base material and exhibits good film strength was formed as a result of the crosslinking reaction of the coating agent proceeding on the base material. Furthermore, it was confirmed that even when the molecular weight of the copolymer used to form the hydrophilic coating film was different as in sample S4 and sample S5, for example, a hydrophilic coating film adhering to base material and exhibiting good film strength can be formed over a wide range of 40,000 to 90,000 weight average molecular weight.

**[Table 2]**

| | Initial Resistance Value (N) | Resistance Value of 50th Measurement (N) |
|---|---|---|
| Sample S1 | 0.118 | 0.294 |
| Sample S2 | 0.049 | 0.078 |
| Sample S3 | 0.078 | 0.245 |
| Sample S4 | 0.039 | 0.059 |
| Sample S5 | 0.059 | 0.088 |
| Sample S6 | 0.353 | 0.667 |
| Sample S7 | 0.006 | 0.706 |

### <Evaluation of adhesion to metal base material>

To evaluate the adhesion to the metal base material, a model using a plate material made of stainless steel was used as the base material instead of the elongated medical devices described in samples S1 to S7. That is, using a plate material made of stainless steel as the metal base material, the adhesion of a coating film formed using a copolymer including the polymerization unit (A) having a cyclic carbonate structure and the polymerization unit (B) having a hydrophilic structure was compared to a polyurethane coating film (comparative example). As the copolymer including the polymerization unit (A) having a cyclic carbonate structure and the polymerization unit (B) having a hydrophilic structure, the same "Poly(MAMCMB-M90G-GCMA) 40:50:10" copolymer as sample S1 was used, and further, the coating film was formed on the substrate under the same conditions as sample S1 using hexamethylenediamine (HMDA) as a curing agent. The polyurethane coating film of the comparative example was formed by a dipping method using Pellethane 2360-80AE (manufactured by Lubrizol Corporation), which is known as a medical polyurethane.

The adhesion to the metal base material was evaluated by a cross-cut test (JIS K5600-5-6, 1999). Specifically, using a cutter knife, incisions were made with respect to the formed coating film in a grid pattern with a 1 mm spacing, and after attaching a transparent adhesive tape and then peeling it off, the state of the grid was observed to confirm the state of peeling of the coating film.

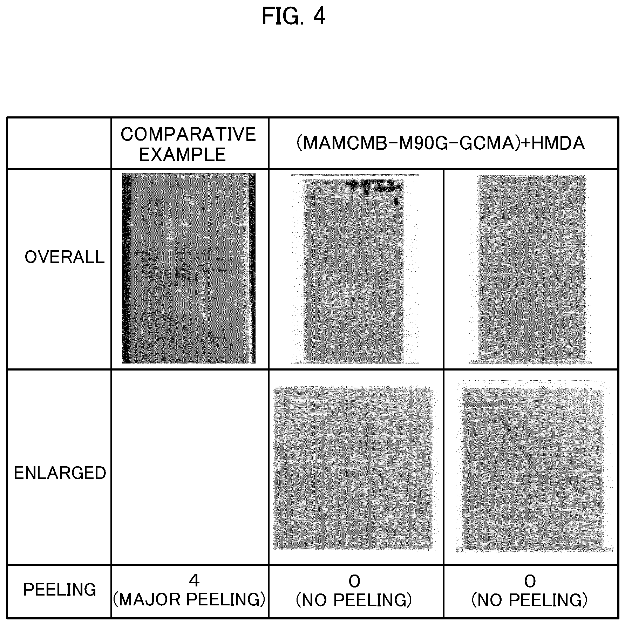

Fig. 4 is an explanatory diagram showing the results of a cross-cut test. As shown in Fig. 4, when the same coating as sample S1 ((MAMCMB-M90G-GCMA) + HMDA) was used, no peeling of the coating film was observed in any part of the grid (evaluation result: 0 (no peeling)). On the other hand, in the coating film of the comparative example, widespread peeling was observed in the tested area (evaluation result: 4 (major peeling)). In this way, it was confirmed that the coating film formed using a coating agent including the polymerization unit (A) having a cyclic carbonate structure and the polymerization unit (B) having a hydrophilic structure exhibited excellent adhesion to the metal base material compared to the coating film of the comparative example, which was formed using a polyol resin and an isocyanate curing agent.

### <Evaluation of adhesion to urethane base material>

The adhesion of the coating film was evaluated by using, as the urethane base material, a guide wire provided with a urethane coating layer on the surface of the metal coil portion (urethane jacket guide wire). Specifically, the adhesion of a coating film formed using a copolymer including the polymerization unit (A) having a cyclic carbonate structure and the polymerization unit (B) having a hydrophilic structure and a coating film formed using a copolymer including a structural unit derived from a monomer containing an epoxy group instead of the polymerization unit (A) having a cyclic carbonate structure were compared. "Poly(MAMCMB-M90G-GCMA) 40:50: 10" was used as the copolymer including the polymerization unit (A) having a cyclic carbonate structure and the polymerization unit (B) having a hydrophilic structure, and the coated guide wire had the same configuration as sample S2 described above. Furthermore, as the copolymer including the structural unit derived from a monomer containing an epoxy group used instead of the polymerization unit (A) having a cyclic carbonate structure, the copolymer "Poly(MAMCMB-M90G-4HBAGE) 40:50:10" including a structural unit derived from 4-hydroxybutyl acrylate glycidyl ether (4HBAGE) was used. The evaluation of the adhesion to the urethane base material was performed using the same method as that already described in "evaluation of film strength".

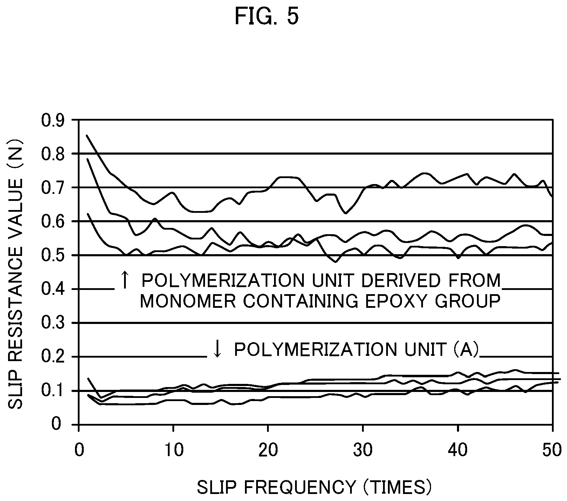

Fig. 5 is an explanatory diagram showing adhesion evaluation results. In Fig. 5, the horizontal axis represents the number of times (slip frequency) the sample was pulled out while measuring the resistance value, and the vertical axis represents the measured resistance value (slip resistance value). Here, three samples were prepared for each of a coated guide wire (labeled as "polymerization unit (A)" in Fig. 5) equivalent to sample S2, which was coated using a copolymer including the polymerization unit (A) having a cyclic carbonate structure, and a coated guide wire (labeled as "polymerization unit derived from monomer containing epoxy group" in Fig. 5) coated using a copolymer including a polymerization unit derived from a monomer containing an epoxy group, and the testing described above was performed for each of the samples. As shown in Fig. 5, it was confirmed when the coating agent including the polymerization unit (A) having a cyclic carbonate structure and the polymerization unit (B) having a hydrophilic structure was used, a hydrophilic coating film is obtained that exhibits excellent adhesion to a urethane base material compared to a case where the coating agent including a polymerization unit derived from a monomer containing an epoxy group and the polymerization unit (B) having a hydrophilic structure was used.

### <Evaluation of catalytic action under low-temperature conditions>

The catalytic action of the polymerization unit (B) was evaluated using various copolymers in which the polymerization unit (B) having a hydrophilic structure was changed, and forming hydrophilic coating films under relatively low-temperature conditions. Specifically, copolymers having, as the polymerization unit (B) having a hydrophilic structure, structural units derived from each of N,N-dimethylacrylamide (DMAAm), N-vinylpyrrolidone (NVP), N-methacryloylaminopropyl-N,N-dimethylammonium-α-N-methylcarboxybetaine (MAMCMB) and methoxyethyl acrylate (MEA) were prepared and evaluated. Each copolymer also contained a structural unit derived from (2-oxo-1,3-dioxolan-4-yl)methyl methacrylate (GCMA) as the polymerization unit (A) having a cyclic carbonate structure. Further, each copolymer contained 10 mol% of the polymerization unit (A) and 90 mol% of the polymerization unit (B). Among the copolymers mentioned above, the copolymer having a structural unit derived from DMAAm as the polymerization unit (B) was the same as the copolymer used in sample S5 described above, and the weight average molecular weight was approximately 40,000. The copolymer having a structural unit derived from NVP as the polymerization unit (B) had a weight average molecular weight of approximately 55,000, the copolymer having a structural unit derived from MAMCMB had a weight average molecular weight of approximately 100,000, and the copolymer having a structural unit derived from MEA had a weight average molecular weight of approximately 40,000.

As the base material on which the hydrophilic coating film was formed, a guide wire provided with a urethane coating layer on the surface of the metal coil portion (urethane jacket guide wire) was used. After preparing each of the copolymers described above, each copolymer was dissolved in dimethylformamide to a concentration of 20 wt%. Then, immediately before being coated on the base material, the coating agent having the copolymer dissolved therein was mixed with a 5% hexamethylene diamine (HMDA) ethanol solution as a crosslinking agent at a weight ratio of 5:3, and was sufficiently dissolved. The coating agent having the crosslinking agent added thereto was coated on the base material by a dip coating method. After coating the coating agent, the change in viscosity was measured after being left to stand for 1 hour, 2 hours, 3 hours, and 4 hours at room temperature (26°C). The viscosity measurement was performed for each coating agent left to stand on the base material as described above using a rotational vibration-type viscometer (VISCOMETER VM-10A-L, manufactured by Sansho Co., Ltd.).

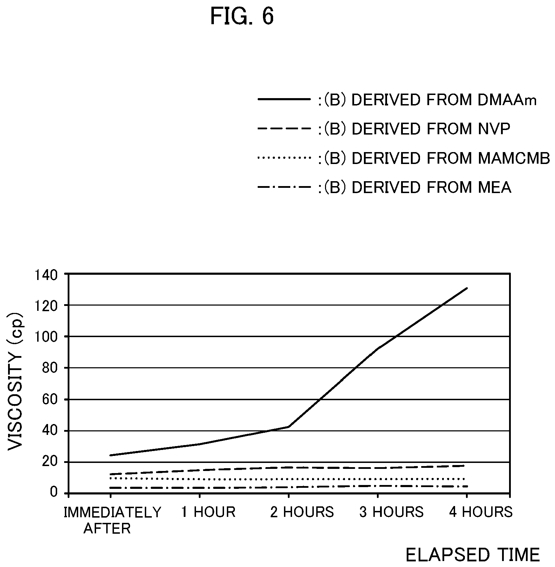

Fig. 6 is an explanatory diagram showing the change in viscosity over time of coating agents containing polymers having different polymerization units (B). In Fig. 6, the copolymers are distinguished by showing the type of the polymerization unit (B). As shown in Fig. 6, the viscosity increased with time in only the case where the coating agent having a structural unit derived from DMAAm was used as the polymerization unit (B), and it was confirmed that crosslinking proceeds even at room temperature. Generally, when a betaine structure provided with a quaternary ammonium or an amide structure provided with a tertiary ammonium is used as the hydrophilic structure of the polymerization unit (B), the quaternary ammonium or the tertiary ammonium is considered to act as a catalyst of the reaction pertaining to the crosslinking of the coating agent. From the results of Fig. 6, and specifically, when the polymerization unit (B) included a structural unit derived from DMAAm, that is, included a tertiary ammonium that does not form a ring structure, a high activity that causes the reaction pertaining to the crosslinking of the coating agent to proceed was confirmed even under low-temperature conditions such as room temperature.

### DESCRIPTION OF REFERENCE NUMERALS

10 Base material

## Claims

1. An elongated medical device, wherein
a coating film is formed on a surface of a base material, and
the coating film is composed of a polymeric material in which a copolymer containing a polymerization unit having a hydrophilic structure has been crosslinked by a structure given by any of formulas (1) to (3).
(1): -CH(OH)-CH(R¹)-O-C(=O)-NH-R²-NH-C(=O)-O-CH(R¹)-CH(OH)-
(2): -CH(OH)-CH(R¹)-O-C(=O)-NH-R²-NH-C(=O)-O-CH(CH(R¹)-OH)-
(3): -CH(CH(R¹)-OH)-O-C(=O)-NH-R²-NH-C(=O)-O-CH(CH(R¹)-OH)-
, wherein R¹ may be the same or different and represents a hydrogen atom, a linear alkyl group having 1 or more carbon atoms, or a branched alkyl group having 1 or more carbon atoms; and R² is an alkylene group having 1 or more carbon atoms, a divalent alicyclic hydrocarbon group containing an alicyclic structure having 3 or more carbon atoms, or a divalent aromatic group containing an aromatic ring structure having 6 or more carbon atoms, and the alkylene group, the alicyclic hydrocarbon group, and the aromatic group may have a divalent group between the carbon atoms represented by - NR³-, where R³ is a hydrogen atom or an alkyl group having 1 to 8 carbon atoms.

2. The elongated medical device according to claim 1, wherein the hydrophilic structure is charge neutral.

3. The elongated medical device according to claim 1 or 2, wherein the hydrophilic structure contains at least one type of structure selected from a group consisting of a betaine structure, an amide structure, a lactam structure, and a polyalkylene oxide structure.

4. The elongated medical device according to claim 3, wherein the hydrophilic structure contains, as a positively charged functional group, at least one of a betaine structure provided with a quaternary ammonium and an amide structure provided with a tertiary ammonium.

5. The elongated medical device according to any one of claims 1 to 4, wherein the base material contains, on a surface of the base material, at least one of a metal, a polymeric material having a group capable of forming a hydrogen bond, and polyurethane.

6. The elongated medical device according to any one of claims 1 to 5, wherein the elongated medical device is a guide wire or a catheter.

## Patentansprüche

1. Längliche medizinische Vorrichtung, wobei
ein Beschichtungsfilm auf einer Oberfläche eines Grundmaterials ausgebildet ist und
der Beschichtungsfilm aus einem polymeren Material zusammengesetzt ist, in dem ein Copolymer, das eine Polymerisationseinheit mit einer hydrophilen Struktur enthält, durch eine Struktur vernetzt wurde, die durch eine der Formeln (1) bis (3) gegeben ist.
(1): -CH(OH)-CH(R¹)-O-C(=O)-NH-R²-NH-C(=O)-O-CH(R¹)-CH(OH)-
(2): -CH(OH)-CH(R¹)-O-C(=O)-NH-R²-NH-C(=O)-O-CH(CH(R¹)-OH)-
(3): -CH(CH(R¹)-OH)-O-C(=O)-NH-R²-NH-C(=O)-O-CH(CH(R¹)-OH)-
, wobei R¹ gleich oder unterschiedlich sein kann und ein Wasserstoffatom, eine lineare Alkylgruppe mit 1 oder mehr Kohlenstoffatomen oder eine verzweigte Alkylgruppe mit 1 oder mehr Kohlenstoffatomen darstellt, und R² eine Alkylengruppe mit 1 oder mehr Kohlenstoffatomen, eine zweiwertige alicyclische Kohlenwasserstoffgruppe, die eine alicyclische Struktur mit 3 oder mehr Kohlenstoffatomen enthält, oder eine zweiwertige aromatische Gruppe, die eine aromatische Ringstruktur mit 6 oder mehr Kohlenstoffatomen enthält, ist, wobei die Alkylengruppe, die alicyclische Kohlenwasserstoffgruppe und die aromatische Gruppe eine zweiwertige Gruppe zwischen den Kohlenstoffatomen aufweisen können, die durch -NR³- dargestellt ist, wobei R³ ein Wasserstoffatom oder eine Alkylgruppe mit 1 bis 8 Kohlenstoffatomen ist.

2. Längliche medizinische Vorrichtung gemäß Anspruch 1, wobei die hydrophile Struktur ladungsneutral ist.

3. Längliche medizinische Vorrichtung gemäß Anspruch 1 oder 2, wobei die hydrophile Struktur mindestens eine Strukturart enthält, ausgewählt aus einer Gruppe bestehend aus einer Betainstruktur, einer Amidstruktur, einer Lactamstruktur und einer Polyalkylenoxidstruktur.

4. Längliche medizinische Vorrichtung nach Anspruch 3, wobei die hydrophile Struktur als positiv geladene funktionelle Gruppe mindestens eine Betainstruktur, die mit einem quaternären Ammonium vorgesehen ist, oder eine Amidstruktur, die mit einem tertiären Ammonium vorgesehen ist, enthält.

5. Längliche medizinische Vorrichtung gemäß einem der Ansprüche 1 bis 4, wobei das Grundmaterial auf einer Oberfläche des Grundmaterials mindestens eines der folgenden Elemente enthält: ein Metall, ein polymeres Material mit einer Gruppe, die eine Wasserstoffbrücke bilden kann, und Polyurethan.

6. Längliche medizinische Vorrichtung gemäß einem der Ansprüche 1 bis 5, wobei die längliche medizinische Vorrichtung ein Führungsdraht oder ein Katheter ist.

## Revendications

1. Dispositif médical allongé, dans lequel
un film de revêtement est formé sur une surface d'un matériau de base, et
le film de revêtement est composé d'un matériau polymère dans lequel un copolymère contenant une unité de polymérisation présentant une structure hydrophile a été réticulé par une structure donnée par l'une quelconque des formules (1) à (3) :
(1) : -CH(OH)-CH(R¹)-O-C(=O)-NH-R²-NH-C(=O)-O-CH(R¹)-CH(OH)-
(2) : -CH(OH)-CH(R¹)-O-C(=O)-NH-R²-NH-C(=O)-O-CH(CH(R¹)-OH)-
(3) : -CH(CH(R¹)-OH)-O-C(=O)-NH-R²-NH-C(=O)-O-CH(CH(R¹)-OH)-
, dans lesquelles R¹ peut être identique ou différent et représente un atome d'hydrogène, un groupe alkyle linéaire présentant 1 ou plusieurs atomes de carbone ou un groupe alkyle ramifié présentant 1 ou plusieurs atomes de carbone ; et R² est un groupe alkylène présentant 1 ou plusieurs atomes de carbone, un groupe hydrocarboné alicyclique divalent contenant une structure alicyclique présentant 3 atomes de carbone ou plus ou un groupe aromatique divalent contenant une structure cyclique aromatique présentant 6 atomes de carbone ou plus, et le groupe alkylène, le groupe hydrocarboné alicyclique et le groupe aromatique peuvent présenter un groupe divalent entre les atomes de carbone représenté par -NR³-, où R³ est un atome d'hydrogène ou un groupe alkyle présentant de 1 à 8 atomes de carbone.

2. Dispositif médical allongé selon la revendication 1, dans lequel la structure hydrophile est de charge neutre.

3. Dispositif médical allongé selon la revendication 1 ou 2, dans lequel la structure hydrophile contient au moins un type de structure sélectionné parmi un groupe consistant en une structure de bétaïne, une structure d'amide, une structure de lactame et une structure d'oxyde de polyalkylène.

4. Dispositif médical allongé selon la revendication 3, dans lequel la structure hydrophile contient, en tant que groupe fonctionnel chargé positivement, au moins l'une parmi une structure de bétaïne pourvue d'un ammonium quaternaire et une structure d'amide pourvue d'un ammonium tertiaire.

5. Dispositif médical allongé selon l'une quelconque des revendications 1 à 4, dans lequel le matériau de base contient, sur une surface du matériau de base, au moins l'un parmi un métal, un matériau polymère présentant un groupe capable de former une liaison hydrogène et un polyuréthane.

6. Dispositif médical allongé selon l'une quelconque des revendications 1 à 5, dans lequel le dispositif médical allongé est un fil-guide ou un cathéter.
